# EUROPÄISCHE PATENTANMELDUNG

(11) **EP 1 310 566 A2**
(43) Veröffentlichungstag der Anmeldung: **14.05.2003**
(21) Anmeldenummer: 02022055.4
(22) Anmeldetag: 02.10.2002
(51) Int. Cl.: C12Q 1/68

(54) **Verfahren zum Identifizieren einer rekombinanten Nucleinsäure**

(30) Priorität: 08.10.2001 DE 20116494 U; 03.07.2002 DE 10229793
(71) Anmelder: Medizinische Hochschule Hannover, 30625 Hannover (DE)
(72) Erfinder: Wilkens, Ludwig, 30539 Hannover (DE)
(74) Vertreter: Eisenführ, Speiser & Partner

(57) **Zusammenfassung**

Die vorliegende Erfindung betrifft ein Verfahren zum Identifizieren einer rekombinanten Nucleinsäure, insbesondere einer mRNA, in einem Nucleinsäuregemisch.

## Beschreibung

Die Erfindung betrifft ein Verfahren zum Identifizieren einer rekombinanten Nucleinsäure, insbesondere einer mRNA, in einem Nucleinsäure-Gemisch.

Durch spontane Umlagerungen, beispielsweise Chromosomen-Translokationen, oder durch in vitro-Manipulationen können Gene entstehen, die Teile von zwei oder mehr verschiedenen Genen umfassen (Fusionsgene). Unter einem Gen wird im Rahmen der vorliegenden Erfindung jeder Abschnitt einer erbinformationtragenden Substanz, insbesondere einer Nucleinsäure, verstanden, der an der Ausprägung eines phänotypischen Merkmals beteiligt ist (auch: Gen im weiteren Sinne). Hierzu zählen insbesondere Gene im engeren Sinne, also solche Abschnitte einer erbinformationtragenden Substanz, namentlich einer Desoxyribonucleinsäure (DNA), die in einer Zelle als Vorlage für die Synthese einer weiteren erbinformationtragenden Substanz, namentlich einer Ribonucleinsäure (RNA), dienen können. Daneben wird hier unter einem Gen im weiteren Sinne auch jeder Abschnitt einer erbinformationtragenden Substanz (insbesondere DNA oder RNA) verstanden, der an der Regulation der Synthese der weiteren erbinformationtragenden Substanz und/oder Proteinen beteiligt ist, insbesondere also Promotoren und andere regulatorische Elemente.

Neben den Veränderungen, bei denen Teile von zwei oder mehr verschiedenen Genen unter Bildung eines neuen Gens zusammengeführt werden, kann es durch Deletionsereignisse dazu kommen, dass ursprünglich weit entfernte Abschnitte eines Gens unter Verlust des zwischen den Abschnitten liegenden Bereichs fusioniert werden. Als Ergebnis beider Arten von Umlagerungen kann das neu gebildete (fusionierte) Gen zu einer Stoffwechselstörung der betroffenen Zelle bzw. des gesamten Organismus führen. Insbesondere können durch solche Umlagerungen Tumore wie beispielsweise die chronische myeloische Leukämie (CML) ausgelöst werden. Es besteht daher ein Bedarf, rekombinante Gene, also solche Gene, die durch Umlagerungen entstanden sind, zu identifizieren.

Herkömmlicherweise werden Umlagerungen durch Färbeverfahren lichtmikroskopisch sichtbar gemacht. Im Falle der chronischen myeloischen Leukämie sind beispielsweise häufig die langen Arme der Chromosomen 9 und 22 umgelagert. Ein Nachweis dieser Umlagerung kann daher einen Hinweis auf die Ursache von CML liefern.

Es gibt jedoch auch Fälle, in denen der Nachweis von Umlagerungen nicht durch Färbeverfahren gelingt, in denen aber dennoch das Vorliegen einer Umlagerung vermutet wird. Zwar sind bei der CML die Sequenzen der beteiligten Genabschnitte bekannt, so dass eine Umlagerung auch mittels der Polymerase-Kettenreaktion (PCR) nachgewiesen werden kann. Dieses Verfahren ist jedoch dann nicht durchführbar, wenn die Sequenzen der beteiligten Genabschnitte noch nicht bekannt sind. Bisher kann die Suche nach Genen, die durch Umlagerung entstanden sind, daher nicht systematisch durchgeführt werden.

Es war daher eine Aufgabe der vorliegenden Erfindung, ein allgemein anwendbares Verfahren anzugeben, mit dem Abschnitte von Genen identifiziert werden können, die durch eine Umlagerung zu einem neuen Gen fusioniert wurden. Das Verfahren sollte ohne vorherige Kenntnis der beteiligten Genabschnitte durchführbar sein.

Die Aufgabe wird erfindungsgemäß gelöst durch ein Verfahren zum Identifizieren einer rekombinanten Nucleinsäure in einem Nucleinsäure-Gemisch, gekennzeichnet durch die Schritte:
a) Bereitstellen eines ersten, zu untersuchenden Nucleinsäure-Gemischs und eines zweiten, mit den Nucleinsäuren des zu untersuchenden Nucleinsäure-Gemischs im wesentlichen hybridisierbaren Nucleinsäure-Vergleichsgemischs, das im wesentlichen frei ist von der zu identifizierenden rekombinanten Nucleinsäure,
b) Inkontaktbringen der beiden Nucleinsäure-Gemische, um eine Hybridisierung von Nucleinsäuren des ersten Nucleinsäure-Gemischs mit Nucleinsäuren des zweiten Nucleinsäure-Gemischs unter Bildung zumindest abschnittweise doppelsträngiger Nucleinsäuren zu ermöglichen, und
c) Entfernen von Nucleinsäuren, soweit wie deren 5'-Enden einzelsträngig sind.

In dem in Schritt b erhaltenen Hybridisierungsgemisch liegen im wesentlichen drei Formen von Nucleinsäure-Hybridisierungsprodukten vor: 1. nicht hybridisierte einzelsträngige Nucleinsäuren und solche Einzelstränge, die abschnittweise mit sich selbst hybridisiert sind (interne Hybridisierung); 2. im wesentlichen vollständig hybridisierte Nucleinsäure-Doppelstränge, wie sie bei Hybridisierungen komplementärer Stränge gewöhnlich entstehen; und 3. abschnittweise doppelsträngige Nucleinsäuren. Rekombinante Nucleinsäuren mit einer Sequenz, die als Ergebnis einer Umlagerung beschrieben werden kann, bilden bei der Hybridisierung mit den jeweils entsprechenden nicht rekombinanten Nucleinsäuren (ursprüngliche Nucleinsäuren) "Y"-förmige Nucleinsäure-Hybridisierungsprodukte (Y-Hybride), also Nucleinsäure-Hybridisierungsprodukte der dritten Form. Die Erfindung ermöglicht nun auf vorteilhaft einfache Weise, diese Y-Hybride gegenüber den Hybridisierungsprodukten der ersten und zweiten Form besonders zu kennzeichnen und gleichzeitig die Hybridisierungsprodukte der ersten Form weitgehend zu beseitigen:

Besitzt eine Nucleinsäure einen am 5'-Ende beginnenden einzelsträngigen, ungepaarten Abschnitt, so wird dieser entfernt. Hierzu kann sich der Fachmann eines Verfahrens seiner Wahl bedienen. Besonders bevorzugt ist es, die Nucleinsäuren von ihrem freien 5'-Ende ausgehend durch Behandlung mit Mung Bean-Nuclease (E.C. Nummer 3.1.30.2) abzubauen.

Vom Abbau sind zum einen die Nucleinsäuren der ersten Form betroffen. Diese sind entweder vollständig einzelsträngig, so dass sie von ihrem 5'-Ende ausgehend vollständig abgebaut werden, oder sie bilden durch Hybridisierung mit sich selbst Schleifen. In diesem Fall werden die Nucleinsäuren vom freien 5'-Ende ausgehend nur bis zum Beginn des doppelsträngigen Abschnitts abgebaut. Häufig sind jedoch interne Hybridisierungen nicht sehr stabil, so dass sich die miteinander hybridisierten Strangabschnitte spontan voneinander lösen, die Nucleinsäure in soweit einzelsträngig und daher weiter abgebaut wird.

Von den Y-Hybriden wiederum wird jeweils der Arm des "Y", der das ungepaarte 5'-Ende umfasst, bis zum Beginn des doppelsträngigen Abschnitts abgebaut. Während der doppelsträngige Abschnitt bei Hybridisierungsprodukten der ersten Form im wesentlichen das Ergebnis einer nur zufälligen abschnittweisen Homologie und daher zumeist recht kurz ist, umfassen die rekombinanten Nucleinsäuren definitionsgemäß Abschnitte aus zwei oder mehr verschiedenen Genen oder ursprünglich voneinander beabstandete Abschnitte eines Gens im engeren Sinne. Daher sind die komplementären Abschnitte der rekombinanten und der entsprechenden nicht rekombinanten Nucleinsäuren häufig länger als die zueinander komplementären Abschnitte von Hybridisierungsprodukten der ersten Form. Dementsprechend ist der doppelsträngige Abschnitt eines Y-Hybrids häufig im Vergleich zu Hybridisierungsprodukten der ersten Form stabiler, so dass der doppelsträngige Abschnitt beständiger gegen den vom 5'-Ende voranschreitenden Abbau ist. Im Ergebnis bestehen somit die Y-Hybride nach Durchführen von Schritt c aus einem doppelsträngigen Abschnitt und dem verbleibenden einzelsträngigen freien 3'-Endabschnitt.

Die nach Durchführen von Schritt c teilweise abgebauten Y-Hybride sind in dem Gemisch der Hybridisierungsprodukte ihrer Struktur nach auffällig. Im wesentlichen werden es nur die teilweise abgebauten Y-Hybride sein, die sowohl einen doppelsträngigen als auch einen einzelsträngigen Nucleinsäureabschnitt umfassen. Die abgebauten Y-Hybride können daher auf vorteilhaft einfache Weise von den übrigen Hybridisierungsprodukten getrennt und analysiert werden. Vorzugsweise werden die abgebauten Y-Hybride durch Elektorphoreseverfahren, insbesondere durch SSCP-Elektrophorese, oder durch Dichtezentrifugation von den übrigen Hybridisierungsprodukten getrennt.

Besonders bevorzugt ist es, wenn beim Inkontaktbringen der beiden Nucleinsäure-Gemische in Schritt b die Konzentration der Nucleinsäuren des zu untersuchenden Nucleinsäuregemischs (erstes Nucleinsäure-Gemisch) die Konzentration der Nucleinsäuren des Vergleichsgemischs (zweites Nucleinsäure-Gemisch) übersteigt. Die Nucleinsäuren des ersten Nucleinsäuregemischs liegen daher, wenn beide Gemische vereinigt werden, gegenüber denen des zweiten Nucleinsäuregemischs im Überschuss vor.

Vorzugsweise werden nach Schritt c Nucleinsäuren in soweit abgebaut, wie sie doppelsträngig sind. Zum Abbau der doppelsträngigen Nucleinsäuren bzw. doppelsträngigen Nucleinsäure-Abschnitte werden vorzugsweise DNAsen wie die Desoxyribonuclease I (E.C. Nummer 3.1.21.1), Desoxyribonuclease II (E.C. Nummer 3.2.22.1) und Desoxyribonuclease V (E.C. Nummer 3.1.11.5) verwendet.

Durch den Abbau doppelsträngiger Nucleinsäuren bzw. doppelsträngiger Nucleinsäure-Abschnitte werden zum einen die Hybridisierungsprodukte der zweiten Form beseitigt, zum anderen werden aus den in Schritt c behandelten Y-Hybriden die nicht hybridisierten, freien Einzelstrangabschnitte gewonnen. Das Beseitigen der Hybridisierungsprodukte der zweiten Art vereinfacht es, die nicht hybridisierten Einzelstrangabschnitte beispielsweise durch Elektrophorese oder Dichtezentrifugation aus einem Reaktionsansatz zu isolieren. Diese nicht hybridisierten Einzelstrangabschnitte folgen in den entsprechenden nicht rekombinanten Nucleinsäuren nicht auf den abgebauten (doppelsträngigen) Nucleinsäureabschnitt, sondern treten aufgrund der Rekombination auf. Anhand der Analyse der Einzelstrangabschnitte kann somit auf die Art der Rekombination geschlossen werden; insbesondere können die an der Rekombination beteiligten Nucleinsäureabschnitte zuverlässig identifiziert werden.

Vorzugsweise werden die so identifizierten Nucleinsäureabschnitte sequenziert. Die erhaltenen Sequenzen können mit bereits bekannten Sequenzen, beispielsweise solchen, die im humanen Genomprojekt gefunden wurden, verglichen werden. Dadurch kann der genaue Ort eines Rekombinationsereignisses, das die Bildung der rekombinanten Nucleinsäure ausgelöst hat, bestimmt werden. Ferner kann ermittelt werden, zu welchem Gen im engeren Sinne die ermittelte Sequenz gehört. Ferner können Rückschlüsse auf die Funktion dieses Gens, beispielsweise für die Tumorbildung und -entwicklung, gezogen werden.

Die Sequenzen können auch zum Herstellen einer Sonde verwendet werden, um die gesamte rekombinante Nucleinsäure und/oder das entsprechende rekombinante Gen im engeren Sinne nachzuweisen und gegebenenfalls zu isolieren. Die rekombinante Nucleinsäure bzw. das rekombinante Gen kann insgesamt sequenziert werden, so dass die Sequenz und Funktion aller in der rekombinanten Nucleinsäure bzw. im rekombinanten Gen vorliegenden Nucleinsäure-Abschnitte ermittelt werden kann. Ferner können Struktur und Funktion eines von dem rekombinanten Gen bzw. der rekombinanten Nucleinsäure codierten Proteins bestimmt werden.

Ferner ist ein Verfahren bevorzugt, bei dem nach Schritt b Nucleinsäuren markiert werden, die ein einzelsträngiges 3'-Ende besitzen. Die Nucleinsäuren können vor, während oder nach Durchführen von Schritt c und vor oder nach dem soeben beschriebenen Abbau doppelsträngiger Nucleinsäuren bzw. doppelsträngiger Nucleinsäure-Abschnitte markiert werden. Die Markierung erleichtert es, die (gegebenenfalls teilweise abgebauten) Y-Hybride nachzuweisen und kann insbesondere zum Isolieren der (gegebenenfalls teilweise abgebauten) Y-Hybride genutzt werden. Die Nucleinsäuren können beispielsweise durch Biotinylieren, Verbinden mit einem Antigen wie beispielsweise Digoxygenin oder, was bevorzugt wird, durch Anhängen zusätzlicher Nucleotide markiert werden.

Werden die Nucleinsäuren vor Durchführen von Schritt c markiert, dann können auch Hybridisierungsprodukte der ersten Form markiert werden. Diese Hybridisierungsprodukte werden jedoch in Schritt c weitgehend abgebaut, so dass sie das Nachweisen und gegebenenfalls das Isolieren der markierten (gegebenenfalls teilweise abgebauten) Y-Hybride nicht wesentlich beeinträchtigen.

Vorzugsweise werden zum Markieren ein oder mehr Thymidinnucleotide an das einzelsträngige 3'-Ende der zu markierenden Nucleinsäure angehängt. Dies kann insbesondere durch Verwenden einer Transcriptase erreicht werden. Das Anhängen zumindest eines Thymidinnucleotids erleichtert es bereits, die Nucleinsäure in einen Vektor mit einem oder mehreren überhängenden Adenosin-Nucleotiden zu klonieren.

Bevorzugt ist es jedoch, mehr als ein Thymidinnucleotid an das einzelsträngige 3'-Ende der zu markierenden Nucleinsäure anzuhängen. Insbesondere ist es bevorzugt, 10 bis 15 Thymidinnucleotide anzuhängen. Dies ist bei Verwendung einer Transcriptase leicht zu erreichen. Die angehängten Thymidinnucleotide können nicht nur das Klonieren in einen Vektor mit überhängenden Adenosin-Nucleotiden erleichtern, sondern auch unmittelbar als Startabschnitt für eine Nucleinsäure-Vervieifältigung, insbesondere eine PCR, dienen. Bei der Nucleinsäure-Vervielfältigung durch eine PCR ist es daher bevorzugt, wenn ein erster Primer einen Abschnitt von zumindest 8 aufeinanderfolgenden Adenosinnucleotiden umfasst,. Ferner ist es bevorzugt, als zweiten Primer ein Gemisch aus Hexanucleotiden mit zufälliger Sequenz zu verwenden. Bei dieser Art Primer ist aus statistischen Gründen anzunehmen, dass ein zu einem der Hexanucleotide komplementärer Abschnitt auf der zu vervielfältigenden Nucleinsäure existiert.

Erfindungsgemäß wird insbesondere ein Verfahren zum Identifizieren einer rekombinanten mRNA angegeben, wobei das Verfahren gekennzeichnet ist durch die Schritte:
a) Bereitstellen eines zu untersuchenden mRNA-Gemischs und eines mRNA-Vergleichsgemischs, das im wesentlichen frei ist von der zu identifizierenden rekombinanten mRNA,
b) Herstellen zweier Gemische einzelsträngiger cDNA, wobei
   - die cDNA des ersten cDNA-Gemischs komplementär ist zur mRNA des zu untersuchenden mRNA-Gemischs und die cDNA des zweiten cDNA-Gemischs der mRNA des mRNA-Vergleichsgemischs in nichtkomplementärer Weise entspricht, oder
   - die cDNA des ersten cDNA-Gemischs der mRNA des zu untersuchenden Gemischs in nichtkomplementärer Weise entspricht und
      die cDNA des zweiten cDNA-Gemischs komplementär ist zur mRNA des mRNA-Vergleichsgemischs,
c) Durchführen eines Verfahrens nach einem der vorherigen Ansprüche mit dem ersten cDNA-Gemisch als erstem Nucleinsäure-Gemisch und dem zweiten cDNA-Gemisch als zweitem Nucleinsäure-Gemisch.

Die Ausprägung neuer phänotypischer Merkmale ist häufig auf eine Mutation eines Gens im engeren Sinne und damit auf ein rekombinantes Protein bzw. eine rekombinante mRNA zurückzuführen. Diese mRNAs sind einfacher zu gewinnen und, da sie größtenteils frei von Introns sind, häufig kürzer als die zugeordneten DNA-Abschnitte, die das jeweils betroffene Gen im engeren Sinne ausmachen. Das Identifizieren einer rekombinanten mRNA bietet somit zahlreiche Vorteile, die zusätzlich zu den zuvor für die Verfahren zum Identifizieren einer Nucleinsäure beschriebenen Vorteilen nutzbar sind.

Ausgehend von einem zu untersuchenden mRNA-Gemisch, in dem die zu identifizierende rekombinante mRNA vermutet wird, und einem diesem entsprechenden mRNA-Vergleichsgemisch, das im wesentlichen frei ist von der zu identifizierenden rekombinanten mRNA, werden zwei cDNA-Gemische hergestellt. Dabei ist die cDNA des einen cDNA-Gemischs komplementär zu der mRNA des zugeordneten mRNA-Gemischs, während die cDNA des anderen cDNA-Gemischs der mRNA des ihr zugeordneten mRNA-Gemischs in nichtkomplementärer Weise entspricht. Es gibt also zwei Möglichkeiten der Beschaffenheit beider Gemische:

Gemäß der ersten Möglichkeit ist die cDNA des ersten cDNA-Gemischs komplementär zur mRNA des zu untersuchenden Gemischs. Im Ergebnis stimmen somit die Basensequenzen der cDNAs des ersten cDNA-Gemischs mit den Basensequenzen der fiktiven Gegenstränge der jeweils zugeordneten mRNAs überein, wobei, wie für cDNAs typisch, die Base Uracil gegen Thymin ausgetauscht ist.

Die cDNA des zweiten cDNA-Gemischs wiederum entspricht der mRNA des Vergleichsgemischs in nichtkomplementärer Weise. Die Basensequenz der cDNAs des zweiten cDNA-Gemischs stimmt also mit derjenigen der jeweils zugeordneten mRNA überein, wobei, wie für cDNAs typisch, die Base Uracil gegen Thymin ausgetauscht ist.

Gemäß der zweiten Möglichkeit entspricht die cDNA des ersten cDNA-Gemischs der mRNA des zu untersuchenden Gemischs in nichtkomplementärer Weise, während die cDNA des zweiten cDNA-Gemischs komplementär ist zur mRNA des mRNA-Vergleichsgemischs.

Für beide Möglichkeiten gilt somit insgesamt, dass das zweite cDNA-Gemisch, bis auf die rekombinante cDNA des ersten cDNA-Gemischs, im wesentlichen hybridisierbar ist mit dem ersten cDNA-Gemisch.

Die Beschaffenheit beider Gemische gemäß der ersten Möglichkeit ist gegenüber der gemäß der zweiten Möglichkeit bevorzugt. Häufig wird die zur Verfügung stehende mRNA-Menge des zu untersuchenden Gemischs geringer sein als die des Vergleichsgemischs. Zur Herstellung eines cDNA-Gemischs, das dem zugeordneten mRNA-Gemischs in nichtkomplementärer Weise entspricht, ist ein Verfahrensschritt mehr notwendig als bei der Herstellung eines cDNA-Gemischs, das komplementär ist zum zugeordneten mRNA-Gemisch. Bei diesem zusätzlichen Verfahrensschritt können Verluste an cDNA auftreten, die bei einer nur begrenzten Menge an zur Verfügung stehender mRNA stark ins Gewicht fallen können.

Besonders bevorzugt ist ein Verfahren, bei dem zum Herstellen der in nichtkomplementärer Weise entsprechenden cDNA in Schritt b:
b1) einzelsträngige cDNA hergestellt wird, die komplementär zu der mRNA des jeweils zugeordneten mRNA-Gemischs ist,
b2) die Gegenstränge der cDNAs aus Schritt b1 hergestellt werden, und
b3) die in Schritt b2 hergestellten Gegenstränge aufgereinigt werden.

Die zur mRNA des zugeordneten mRNA-Gemischs komplementäre cDNA wird vorzugsweise durch reverse Transkription hergestellt. Zum Herstellen der jeweiligen Gegenstränge der cDNAs aus Schritt b1 kann der Fachmann insbesondere eine DNA-Polymerase verwenden. Die beiden cDNA-Stränge können beispielsweise durch SSCP-Gelelektrophorese voneinander getrennt und der Gegenstrang aufgereinigt werden. Vorzugsweise wird zwischen den Schritten b1 und b3 die RNA durch eine RNAse abgebaut.

Bevorzugt ist es jedoch, wenn die cDNA aus Schritt b1 während oder nach ihrer Herstellung mit einem Marker versehen wird. Dies kann beispielsweise dadurch erfolgen, dass bei der Herstellung einzelsträngiger cDNA in Schritt b1 markierte Nucleotide, beispielsweise biotin- oder digoxygeninmarkierte Nucleotide, verwendet werden, so dass der hergestellte cDNA-Einzelstrang markiert ist. Die Markierung kann auch nach Abschluß der Synthese am cDNA-Einzelstrang angebracht werden. Der in Schritt b2 hergestellte Gegenstrang wird nicht markiert.

Zum Aufreinigen der in Schritt b2 hergestellten Gegenstänge können insbesondere die markierten cDNA-Stränge mit ihrem Marker an einem festen Träger gebunden und der jeweils zugeordnete Gegenstrang durch Elution aufgereinigt werden. Als fester Träger ist insbesondere eine Affinitätsmatrix geeignet. Wurde die cDNA aus Schritt b1 biotinmarkiert, so ist es zum Aufreinigen der in Schritt b2 hergestellten Gegenstränge besonders bevorzugt, wenn der feste Träger Streptavidin umfasst; wurde die cDNA aus Schritt b1 digoxygeninmarkiert, so umfasst der feste Träger vorzugsweise einen Antikörper oder ein Fab-Fragment gegen Digoxygenin.

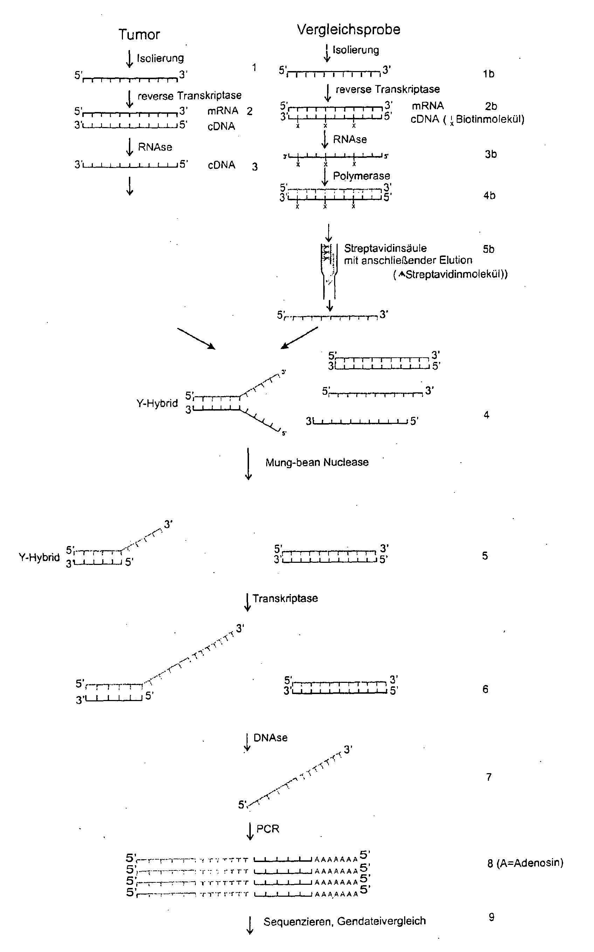

Die Erfindung wird nachfolgend anhand eines Ausführungsbeispiels und der Zeichnung näher beschrieben.

Das Verfahren beginnt mit dem Isolieren von mRNA aus einem Tumor (vgl. Zeichnung, Schritt 1). Die mRNA wird mit einer reversen Transkriptase zu cDNA übersetzt. (vgl. Zeichnung, Schritt 2) Anschließend wird die RNA mit einer RNAse abgebaut (vgl. Zeichnung, Schritt 3), so dass ein erstes cDNA-Gemisch erhalten wird, das im wesentlichen frei von RNA ist.

Zum Herstellen des zweiten cDNA-Gemischs wird mRNA aus einem dem Tumor entsprechenden, jedoch nicht entarteten Gewebe aufgereinigt (vgl. Zeichnung, Schritt 1b) und mit einer reversen Transkriptase zu cDNA übersetzt. Bei der Synthese der cDNA werden biotinylierte Nucleotide als Marker (in der Zeichnung durch "x" angedeutet) verwendet, so dass die synthetisierten cDNA-Stränge biotinmarkiert sind (vgl. Zeichnung, Schritt 2b). Dies entspricht dem oben allgemein beschriebenen Schritt b1.

Anschließend wird die RNA mit einer RNAse abgebaut (vgl. Zeichnung, Schritt 3b), dann werden die biotinylierten Nucleotide ausgewaschen. Im Anschluß hieran werden die Gegenstränge der soeben synthetisierten cDNAs mit einer DNA-Polymerase hergestellt, wobei die verwendeten Nucleotide nicht biotinyliert sind (vgl. Zeichnung, Schritt 4b). Damit ist der zuvor allgemein beschriebene Schritt b2 abgeschlossen.

Zum Aufreinigen der soeben entsprechend Schritt b2 hergestellten cDNA-Gegenstränge wird das Gemisch aus markierten und nicht markierten cDNA-Strängen auf eine Streptavidin-beschichtete Waschsäule gegeben. Das Streptavidin bindet die biotinylierten cDNA-Stränge. Die nicht biotinylierten cDNA-Gegenstränge werden durch Zugabe einer Waschlösung eluiert (vgl. Zeichnung, Schritt 5b). Damit ist auch der zuvor allgemein beschriebene Schritt b3 und damit die Herstellung des zweiten cDNA-Gemischs abgeschlossen.

Nach dem Herstellen des ersten und zweiten cDNA-Gemischs werden beide Nucleinsäuregemische miteinander in Kontakt gebracht, um eine Hybridisierung von cDNAs des ersten cDNA-Gemischs mit cDNAs des zweiten cDNA-Gemischs unter Bildung zumindest abschnittweise doppelsträngiger Nucleinsäuren zu ermöglichen (vgl. Zeichnung, Schritt 4). Dazu werden die cDNA-Gemische miteinander vereinigt; wobei die cDNAs des ersten cDNA-Gemischs im Überschuss vorliegen. Die zueinander komplementären Nucleinsäuren bzw. Nucleinsäure-Abschnitte des ersten und zweiten cDNA-Gemischs können sich dann aneinander anlagern (hybridisieren). Es kommt dabei zur Bildung dreier Formen von Hybridisierungsprodukten (vgl. Zeichnung, Schritt 4): Zum einen verbleiben nicht hybridisierte Einzelstränge, zweitens bilden sich miteinander im wesentlichen vollständig hybridisierte Doppelstränge, und als dritte Form bilden sich Y-förmige Hybridisierungsprodukte (Y-Hybride). Die Y-Hybride werden insbesondere dadurch gebildet, dass eine rekombinante cDNA (also eine cDNA, die aus einer rekombinanten mRNA hervorgegangen ist) des ersten cDNA-Gemischs mit einem komplementären Abschnitt einer cDNA des zweiten cDNA-Gemischs hybridisiert. Da sich in der rekombinanten cDNA an den komplementären Abschnitt ein weiterer Nucleinsäure-Abschnitt anschließt, der in der nicht rekombinanten Nucleinsäure an dieser Stelle nicht vorkommt, kann dieser weitere Nucleinsäure-Abschnitt nicht mit der cDNA des zweiten cDNA-Gemischs hybridisieren. In soweit bleiben daher beide cDNAs einzelsträngig.

Durch Zugabe von Mung bean-Nuclease (vgl. Zeichnung, Schritt 5) werden die Nucleinsäuren abgebaut, soweit sie - ausgehend von ihrem jeweiligen 5'-Ende - einzelsträngig vorliegen. Auf diese Weise werden sowohl einzelsträngige cDNAs als auch die freien 5'-seitigen "Arme" der Y-Hybride abgebaut.

Anschließend wird zum Markieren von cDNAs, die ein einzelsträngiges 3'-Ende besitzen, eine Transkriptase zugegeben. Diese hängt an die freien 3'-Enden der cDNAs eine längere Reihe von Thymidinnucleotiden an. Auf diese Weise können etwa 8 bis 15 Thymidinnucleotide angehängt werden (vgl. Zeichnung, Schritt 6). Die neben den teilweise abgebauten Y-Hybriden noch vorliegenden cDNA-Doppelstränge werden nicht markiert.

Nach dem Markieren werden die cDNAs soweit abgebaut, wie sie doppelsträngig sind. Dazu wird eine DNAse zu den cDNAs zugegeben (vgl. Zeichnung, Schritt 7). Unter der Wirkung der DNAse werden alle doppelsträngigen Nucleinsäuren und doppelsträngigen Nucleinsäure-Abschnitte abgebaut. Die einzelsträngigen Nucleinsäureabschnitte der zuvor bereits teilweise abgebauten Y-Hybride werden durch die DNAse jedoch nicht abgebaut. Nach der DNAse-Behandlung bleiben sie als im wesentlichen einzige Nucleinsäuren mit mehr als 10 Nucleotiden Länge übrig.

Die so erhaltenen cDNA-Abschnitte werden in einem PCR-Verfahren vervielfältigt (vgl. Zeichnung, Schritt 8). Dabei wird der erste PCR-Primer so gewählt, dass er an der am 3'-Ende der cDNA-Abschnitte angeordneten Reihe von Thymidinnucleotiden hybridisieren kann. Als zweiter PCR-Primer wird ein Hexanucleotid-Gemisch gewählt, bei dem aus statistischen Gründen zu erwarten ist, dass eines der Hexanucleotide an einer cDNA hybridisieren kann. Anschließend werden die vervielfältigten cDNA-Abschnitte sequenziert.

## Patentansprüche

1. Verfahren zum Identifizieren einer rekombinanten Nucleinsäure in einem Nucleinsäure-Gemisch, **gekennzeichnet durch** die Schritte:
a) Bereitstellen eines ersten, zu untersuchenden Nucleinsäure-Gemischs und eines zweiten, mit den Nucleinsäuren des zu untersuchenden Nucleinsäure-Gemischs im wesentlichen hybridisierbaren Nucleinsäure-Vergleichsgemischs, das im wesentlichen frei ist von der zu identifizierenden rekombinanten Nucleinsäure,
b) Inkontaktbringen der beiden Nucleinsäure-Gemische, um eine Hybridisierung von Nucleinsäuren des ersten Nucleinsäure-Gemischs mit Nucleinsäuren des zweiten Nucleinsäure-Gemischs unter Bildung zumindest abschnittweise doppelsträngiger Nucleinsäuren zu ermöglichen, und
c) Entfernen von Nucleinsäuren, soweit wie deren 5'-Enden einzelsträngig sind.

2. Verfahren nach Anspruch 1, **dadurch gekennzeichnet, dass** nach Schritt c Nucleinsäuren soweit abgebaut werden, wie sie doppelsträngig sind.

3. Verfahren nach einem der vorherigen Ansprüche, **dadurch gekennzeichnet, dass** nach Schritt b Nucleinsäuren markiert werden, die ein einzelsträngiges 3'-Ende besitzen.

4. Verfahren nach Anspruch 3, **dadurch gekennzeichnet, dass** zum Markieren ein oder mehr Thymidinnucleotide an das einzelsträngige 3'-Ende angehängt werden.

5. Verfahren zum Identifizieren einer rekombinanten mRNA, **gekennzeichnet durch** die Schritte:
a) Bereitstellen eines zu untersuchenden mRNA-Gemischs und eines mRNA-Vergleichsgemischs, das im wesentlichen frei ist von der zu identifizierenden rekombinanten mRNA,
b) Herstellen zweier Gemische einzelsträngiger cDNA, wobei
- die cDNA des ersten cDNA-Gemischs komplementär ist zur mRNA des zu untersuchenden mRNA-Gemischs und
die cDNA des zweiten cDNA-Gemischs der mRNA des mRNA-Vergleichsgemischs in nichtkomplementärer Weise entspricht, oder
- die cDNA des ersten cDNA-Gemischs der mRNA des zu untersuchenden Gemischs in nichtkomplementärer Weise entspricht und
die cDNA des zweiten cDNA-Gemischs komplementär ist zur mRNA des mRNA-Vergleichsgemischs,
c) Durchführen eines Verfahrens nach einem der vorherigen Ansprüche mit dem ersten cDNA-Gemisch als erstem Nucleinsäure-Gemisch und dem zweiten cDNA-Gemisch als zweitem Nucleinsäure-Gemisch.

6. Verfahren nach Anspruch 5, **dadurch gekennzeichnet, dass**
- die cDNA des ersten cDNA-Gemischs komplementär ist zur mRNA des zu untersuchenden mRNA-Gemischs und
- die cDNA des zweiten cDNA-Gemischs der mRNA des mRNA-Vergleichsgemischs in nichtkomplementärer Weise entspricht.

7. Verfahren nach einem der Ansprüche 5 bis 6, **dadurch gekennzeichnet, dass** zum Herstellen der in nichtkomplementärer Weise entsprechenden cDNA in Schritt b:
b1) einzelsträngige cDNA hergestellt wird, die komplementär zu der mRNA des jeweils zugeordneten mRNA-Gemischs ist,
b2) die Gegenstränge der cDNAs aus Schritt b1 hergestellt werden, und
b3) die in Schritt b2 hergestellten Gegenstränge aufgereinigt werden.

8. Verfahren nach Anspruch 7, **dadurch gekennzeichnet, dass** die cDNA aus Schritt b1 während oder nach ihrer Herstellung mit einem Marker versehen wird.

9. Verfahren nach Anspruch 8, **dadurch gekennzeichnet, dass** die markierte cDNA mit ihrem Marker an einem festen Träger gebunden und der Gegenstrang durch Elution aufgereinigt wird.
